# FASCICULE DE BREVET EUROPEEN

(11) **EP 2 378 991 B1**
(45) Date de publication et mention de la délivrance du brevet: **17.10.2012**
(21) Numéro de dépôt: 10706281.2
(22) Date de dépôt: 05.01.2010
(51) Int. Cl.: A61B 17/70

(54) **IMPLANT DYNAMIQUE DE TYPE «VIS TIGE» POUR STABILISER UN RACHIS**
DYNAMISCHES STANGENSCHRAUBENIMPLANTAT ZUR STABILISIERUNG DER WIRBELSÄULE
"ROD SCREW" DYNAMIC IMPLANT FOR STABILIZING A VERTEBRAL COLUMN

(30) Priorité: 07.01.2009 FR 0950068
(43) Date de publication de la demande: 26.10.2011
(73) Titulaire: Creaspine, 33600 Pessac (FR)
(72) Inventeur: VINCENT-PRESTIGIACOMO, Philippe, F-33680 Lacanau-Ocean (FR); ROTH, Nicolas, F-33800 Bordeaux (FR)
(74) Mandataire: Chevalier, Renaud Philippe
(86) Numéro de dépôt international: PCT/FR2010/050007
(87) Numéro de publication internationale: WO 2010/079297

(56) Documents cités:
- US-A1- 2004 049 190
- US-A1- 2008 262 554

## Description

La présente invention se rapporte au domaine de la chirurgie du rachis du corps humain ou animal, et plus particulièrement aux implants pour la stabilisation d'une partie du rachis.

Le rachis, ou colonne vertébrale, a un rôle essentiel à bien des égards pour le corps humain ou animal et notamment, il doit pouvoir absorber des contraintes mécaniques dues aux mouvements du corps. Il arrive cependant que le rachis ne puisse plus assurer correctement ses fonctions, par exemple, à la suite d'un traumatisme ou encore pour des raisons liées à l'âge ou à des maladies qui peuvent induire une usure, ou à la dégénération d'une ou plusieurs articulations des vertèbres : on parle alors d'instabilités. De telles instabilités sont très souvent sources de douleurs et peuvent être traitées par médications, rééducations, ou interventions chirurgicales avec ou sans instrumentalisation.

L'instrumentalisation du rachis consiste à consolider la stabilité du rachis par la pose d'un implant reliant les corps vertébraux d'au moins deux vertèbres consécutives. Classiquement, un tel implant comprend deux éléments liées aux corps vertébraux de type vis, crochets ou câbles et sont reliés entre eux par des éléments de type barre ou plaque. Pour des raisons cliniques, il est souhaitable qu'un tel implant ait une certaine souplesse afin d'absorber des chocs ou pour se déformer et éviter de traumatiser le rachis. Ainsi, la demande de brevet US 6 966 910 décrit un pont intervertébral comportant deux vis reliées par un élément de connexion dont la forme géométrique est conformée pour fléchir en cas de contrainte excessive. Un tel pont intervertébral ne présente cependant pas toute la souplesse necessaire pour ne pas gêner la mobilité de la partie du rachis ainsi traitée. Afin de conférer plus de mobilité à la partie implantée d'un rachis la demande de brevet US 2004/0049190 décrit un dispositif dynamique 1, représenté à la Figure 1 ci-annexée, comportant deux éléments d'ancrage 2 destinés à être fixés à des vertèbres 3 reliés par une barre 4 montée coulissante pour permettre un mouvement dans la direction de la double flèche. Des moyens élastiques 5 sont disposés sur la barre 4, dans l'espace 6 entre les deux éléments d'ancrage 2, symbolisé par la zone carrée en pointillés, afin d'exercer une force dans la direction de l'axe longitudinal de la barre 4. Un tel dispositif dynamique 1 permet une mobilité acceptable de la partie du rachis traitée mais est cependant encombrant et ne peut pas être utilisé pour corriger tous les défauts du rachis. En effet, vu de face ou de dos un rachis sain est sensiblement rectiligne. Par contre, dans un plan sagittal, le rachis présente quatre courbures qui, de bas en haut, sont appellées courbure sacrée, lordose lombaire, cyphose dorsale et lordose cervicale. Or, au niveau de la lordose lombaire, les espaces entre les corps vertébraux sont particulièrement réduits et donc un dispositif dynamique 1 tel que celui décrit dans la demande de brevet US 2004/0049190 ne peut y être positionné à cause de l'encombrement des ses moyens élastiques 5. Par ailleurs, un tel dispositif dynamique 1 ne permet pas un contrôle dynamique du mouvement de rotation de la barre 4 par rapport aux éléments d'ancrage 2.

La présente invention a pour objet de remédier en tout ou partie aux différents inconvénients cités précédemment. Dans ce contexte technique, un but de la présente invention est de fournir un implant pour la stabilisation d'un rachis permettant une mobilité en translation et/ou rotation de la partie du rachis traitée. Plus particulièrement, la présente invention vise à obtenir un implant apte à être implanté au niveau d'une lordose lombaire ou sur une partie d'un rachis dont les vertébres sont très peu espacées.

A cet effet, la présente invention se rapporte à un élément de fixation pour un implant de maintien d'une partie d'un rachis de l'homme ou de l'animal, comprenant, d'une part, un organe d'ancrage destiné à assurer la fixation de l'élément dans un corps vertébral et, d'autre part, une tête dans laquelle est ménagé un orifice de passage conformé pour recevoir une tige de liaison, cet élément étant remarquable en ce qu'il comporte des moyens de centrage de ladite tige de liaison montés sur des moyens de rappel élastique disposés dans ladite tête, lesdits moyens de centrage et ladite tête étant mobiles l'un par rapport à l'autre.

La présente invention se rapporte également à une tige de liaison pour un implant de maintien du rachis de l'homme ou de l'animal, apte à être insérée dans un orifice de passage d'un élément de fixation et comprenant au moins une seconde surface de centrage comportant une encoche et/ou une saillie.

Ainsi, un un élément de fixation selon l'invention, en coopération avec une tige de liaison selon l'invention permet d'obtenir un implant selon l'invention, tel que décrit ci-après, offrant une mobilité adéquate et permettant un contrôle dynamique du déplacement des vertébres d'une partie d'un rachis reliées par ledit implant : en effet, lors d'un déplacement relatif de deux vertébres reliées par un implant selon l'invention, la tige de liaison et l'élément de fixation selon l'invention peuvent bouger en translation et/ou rotation l'un par rapport à l'autre, lesdits moyens de centrage tendant à ramener la tige de liaison et l'élément de fixation dans une position prédéfinie, grâce aux forces de rappel exercées par les moyens de rappel élastique. Les moyens de rappel élastique étant disposés dans la tête de l'élément de fixation, cela permet un positionnement de l'implant selon l'invention au niveau d'une lordose lombaire d'un rachis : en effet, contrairement à un dispositif classique il n'est pas nécessaire de prévoir un espace tel que celui habituellement requis pour les moyens élastiques d'un dispositif dynamique de l'art antérieur.

Dans un mode de réalisation de l'invention, le mouvement relatif des moyens de centrage s'effectue selon un axe transversal, notamment perpendiculaire, à l'axe de l'orifice de passage. Un axe transversal à l'axe de l'orifice permet de réduire l'espace minimal nécessaire entre deux vertébres pour qu'elles puissent être reliées par un implant selon la présente invention.

Dans un mode de réalisation de l'invention, lesdits moyens de rappel élastique tendent à pousser lesdits moyens de centrage vers l'intérieur de l'orifice de passage. Les moyens de centrage poussés vers l'intérieur de l'orifice de passage sont destinés à venir en appui sur la tige de liaison et minimiser le jeu fonctionnel entre l'élément de fixation et ladite tige.

Dans un mode de réalisation de l'invention, ladite tête comporte en outre un logement débouchant dans l'orifice de passage à l'intérieur duquel sont ménagés les moyens de rappel élastique et les moyens de centrage.

Dans un mode de réalisation de l'invention, ledit élément de fixation comprenant en outre un verrou destiné à obturer le logement réalisé débouchant en outre à l'extérieur de ladite tête, le verrou comprenant les moyens de rappel élastique et les moyens de centrage. Un verrou comprenant les moyens de rappel élastique et les moyens de centrage permet de simplifier l'assemblage de l'élément de fixation.

Dans un mode de réalisation de l'invention, le verrou comporte des ailettes souples destinées à coopérer avec au moins une butée ménagée dans la surface du logement. Les ailettes sont simples à réaliser et peuvent être moulées avec le verrou ou bien encore usinées sur le verrou : cela permet de simplifier l'assemblage, le nombre de pièces nécessaire à l'assemblage et les coûts de production de l'élément de fixation selon l'invention.

Dans un mode de réalisation de l'invention, les moyens de centrage sont solidaires de l'organe d'ancrage, lesdits moyens de rappel élastique étant montés entre la tête et ledit organe d'ancrage. Les moyens de rappel élastique peuvent alors être constitués par exemple d'un joint annulaire peut coûteux.

Dans un mode de réalisation de l'invention, la tête et l'organe d'ancrage sont mobiles en translation et/ou en rotation l'un par rapport à l'autre. Ainsi, un implant réalisé à l'aide d'un élément de fixation selon l'invention présente une mobilité accrue.

Dans un mode de réalisation de l'invention, les moyens de rappel élastique sont conçus de manière à exercer un couple de rappel en rotation entre la tête et l'organe d'ancrage. Ainsi, un élément de fixation selon l'invention permet un contrôle dynamique lors de déplacements des vertèbres en translation et/ou en rotation.

Dans un mode de réalisation de l'invention, ledit moyen de centrage comporte une première surface de centrage en regard de l'orifice de passage présentant au moins une pente, notamment de forme plane ou incurvée.

Dans un mode de réalisation de l'invention, la première surface de centrage comporte une encoche.

Dans un mode de réalisation de l'invention, la première surface de centrage comporte un bossage.

Dans un mode de réalisation de l'invention, au moins une partie de la surface délimitant l'orifice de passage présente une surface de centrage supplémentaire comportant un bossage et/ou une encoche.

Dans un mode de réalisation de la tige de liaison selon l'invention, chaque seconde surface de centrage est ménagée à proximité d'une des extrémités de la tige de liaison.

Enfin, la présente invention se rapporte à un implant pour la stabilisation d'un rachis pour le corps humain ou animal comportant au moins un élément de fixation selon l'invention et au moins une tige de liaison selon l'invention insérée dans l'orifice de passage dudit élément de fixation, les moyens de centrage étant conçus pour coopérer avec ladite seconde surface de centrage. De préférence, ladite tige de liaison est mobile en rotation et/ou en translation par rapport à l'organe d'ancrage, lesdites première et seconde surfaces de centrage étant conçues pour coopérer afin de déplacer ladite tige de liaison en rotation et/ou en translation par rapport à l'organe d'ancrage.

L'invention sera mieux comprise à l'aide de la description détaillée qui est exposée ci-dessous en regard des dessins annexés dans lesquels :
la figure 1 représente une vue schématique d'un dispositif dynamique de l'art antérieur, tel que mentionné dans le préambule de la présente description ;
la figure 2 représente une vue schématique d'un implant selon l'invention ;
les figures 3a et 3b représentent des vues schématiques en coupe longitudinale d'un élément de fixation selon un premier mode de réalisation de l'invention et d'une partie d'une tige de liaison selon l'invention ;
les figures 4a à 4c représentent des vues schématiques en coupe longitudinale d'un élément de fixation selon un seconde mode de réalisation de l'invention et d'une partie d'une tige de liaison selon l'invention ;
les figures 5a et 5b représentent des vues schématiques en coupe longitudinale d'un élément de fixation selon un troisième mode de réalisation de l'invention et d'une partie d'une tige de liaison selon l'invention ;
les figures 6a à 6c représentent des vues schématiques en perspectives (6a, 6b) et en coupe longitudinale (6c) d'un élément de fixation selon un quatrième mode de réalisation de l'invention et d'une partie d'une tige de liaison selon l'invention ;
les figures 7a à 7f représentent des vues schématiques en perspective de détails de secondes surfaces de centrage de tige de liaison selon l'invention.

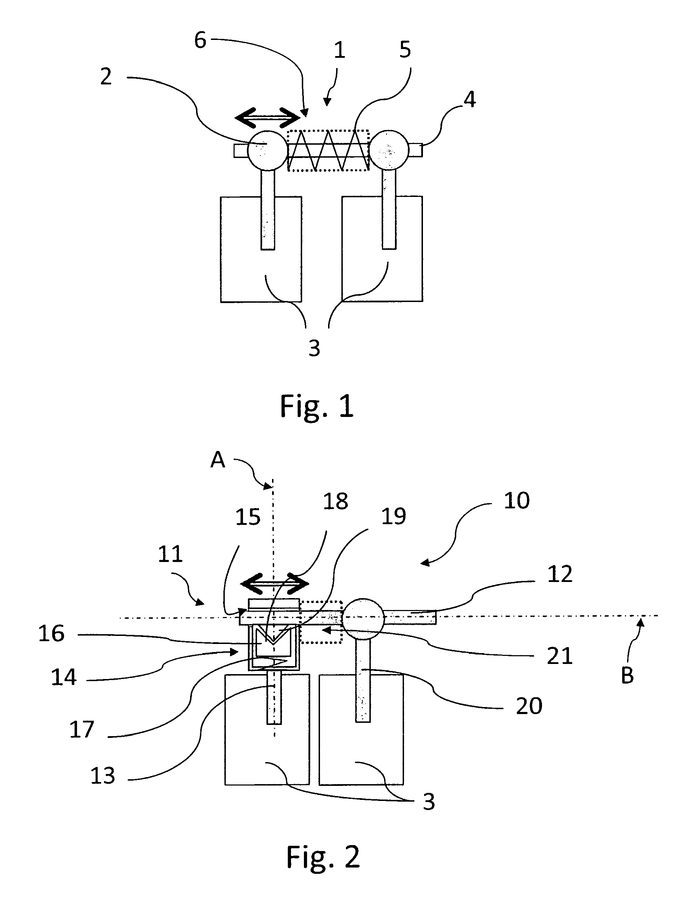

Un implant 10 selon l'invention, tel que représenté de façon schématique sur la Figure 2, comporte au moins un élément de fixation 11 selon l'invention destiné à être ancré dans une vertèbre 3 et au moins une tige de liaison 12 selon l'invention. L'élément de fixation 11 comporte un organe d'ancrage 13 destiné à assurer la fixation dudit élément de fixation 11 dans le corps vertébral d'une vertèbre 3. L'élément de fixation 11 comporte également une tête 14 dans laquelle est ménagé un orifice de passage 15 conformé pour recevoir la tige de liaison 12, de façon à ce que celle-ci puisse coulisser à l'intérieur de cet orifice de passage 15 au moins dans la direction symbolisée par la double flèche. Dans la tête 14 de l'élément de fixation 11 sont disposés des moyens de centrage 16 de ladite tige de liaison 12 montés sur des moyens de rappel élastique 17. Les moyens de centrage 16 présentent une première surface de centrage 18 en regard de l'orifice de passage 15 et présentant au moins une pente plane ou incurvée, ladite première surface de centrage 18 formant un bossage et/ou une encoche. Les moyens de rappel élastique 17 exercent des efforts sur les moyens de centrage 16 de façon à pousser ladite première surface de centrage 18 vers l'intérieur de l'orifice de passage 15.

La tige de liaison 12 présente, à une de ses extrémités destinée à être insérée dans l'orifice de passage 15, une seconde surface de centrage 19 comportant également une encoche et/ou une saillie. A l'autre extrémité de la tige de liaison 12 est fixée, par exemple, une vis d'ancrage 20 classique, destinée à assurer la fixation de l'implant 10 selon l'invention dans une seconde vertèbre 3.

Lorsque la tige de liaison 12 est insérée dans l'orifice de passage 15, les première et seconde surfaces de centrage (18, 19) coopèrent, comme cela est détaillé plus loin, pour déplacer ladite tige de liaison 12, en rotation et/ou en translation, par rapport à l'organe d'ancrage 13.

L'incorporation des moyens de rappel élastique 17 à l'intérieur de la tête 14 permet de libérer l'espace qu'occupaient les moyens élastiques 5 sur la barre 4 d'un dispositif dynamique 1 classique. Ainsi, l'espace 21, symbolisé par la zone carrée en pointillés sur la Figure 2, situé entre l'élément de fixation 11 et la vis d'ancrage 20 de l'implant 10 de la figure 2 est plus restreint que l'espace 6 occupé par les moyens élastiques 5 d'un dispositif dynamique 1 classique représenté à la Figure 1. Par ailleurs, le déplacement relatif des moyens de centrage 16 selon un axe A transversal par rapport à l'axe B de l'orifice de passage 15 permet de positionner les moyens de centrage 16 en dehors de l'espace 21 et donc de réduire encore ce dernier. L'encombrement de l'espace 21 par les moyens de centrage 16 est minimal lorsque l'axe A transversal est perpendiculaire à l'axe B de l'orifice de passage 15.

Un tel espace 21 restreint permet de positionner un implant 10 selon l'invention sur deux vertèbres 3 consécutives situées dans une zone du rachis, telle que la lordose lombaire, où un dispositif dynamique 1 classique ne peut être positionné car trop encombrant.

Dans la suite de la description, et afin d'en simplifier la lecture, des éléments similaires ou communs aux différents éléments de fixation pourront conserver la même numérotation.

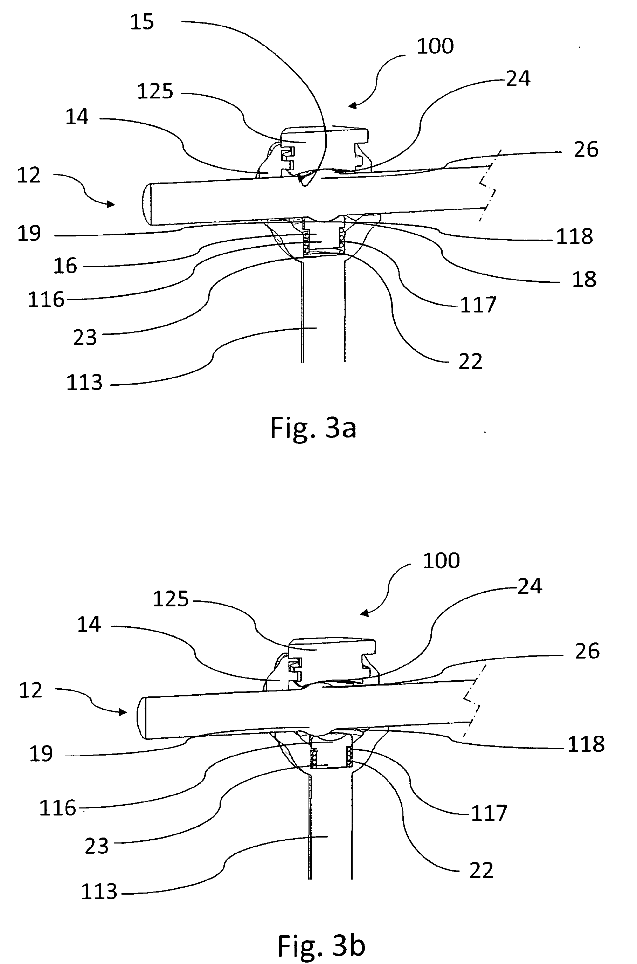

Les Figures 3a et 3b sont des vues en coupe longitudinale d'un élément de fixation 100 selon un premier mode de réalisation de l'invention, dans l'orifice de passage 15 duquel est introduite une tige de liaison 12 selon l'invention. L'élément de fixation 100 présente une tête 14 venant de matière avec l'organe d'ancrage 13 constitué par exemple d'une vis 113. Dans ce mode de réalisation, la tête 14 comporte un logement 22, débouchant à l'intérieur de l'orifice de passage 15, dans lequel sont disposés, d'une part, les moyens de rappel élastique 17, constitués, dans ce cas, d'un ressort hélicoïdal 117, et, d'autre part, les moyens de centrage 17 constitués alors d'un bloc de centrage 116 monté sur le ressort hélicoïdal 117. Le bloc de centrage 116 est mobile suivant un axe parallèle à l'axe longitudinal de la vis 113 et le ressort hélicoïdal 117 pousse le bloc de centrage 116 vers l'intérieur de l'orifice de passage 15 en libérant un espace 23 entre le bloc de centrage 116 et le fond du logement 22.

Le bloc de centrage 116 comporte une première surface de centrage 18, par exemple une encoche sphérique 118, située en regard de l'orifice de passage 15. De façon avantageuse, une surface supplémentaire de centrage 24 délimitant l'orifice de passage 15, et opposée à la première surface de centrage 18, présente également un bossage ou une encoche. Dans le mode de réalisation de l'invention représenté à la Figure 3a, l'évidement 22 se prolonge à travers l'orifice de passage 15 et débouche à l'extérieur de la tête 14. Un tel évidement 22 est simple à réaliser et peut être obturé par un verrou 125. La surface supplémentaire de centrage 24 est, par exemple, la face du verrou 125 destinée à venir en regard de l'orifice de passage 15.

La tige de liaison 12 selon l'invention présente une seconde surface de centrage 19 représentée dans ce mode de réalisation par un bossage arrondi 26. Les première et seconde surfaces de centrage (18, 19) sont maintenues en contact grâce au ressort hélicoïdal 117. En l'absence de sollicitation de la tige de liaison 12 et/ou de l'élément de fixation 100, et grâce aux formes complémentaires du bossage arrondi 26 et de l'encoche sphérique 118, la tige de liaison 12 occupe une position prédéfinie telle que représentée à la Figure 3a.

Lorsqu'une contrainte est appliquée sur l'implant 10 selon l'invention, par exemple lorsque les vertèbres 3 supportant l'implant 10 se déplacent l'une par rapport à l'autre, la tige de liaison 12 et l'élément de fixation 100 peuvent être amenés à se déplacer l'un par rapport à l'autre. Durant un tel déplacement les première et seconde surfaces de centrage (18, 19) glissent l'une sur l'autre et sont maintenues en contact grâce à la pression exercée par le ressort hélicoïdal 117. Lors du déplacement en translation de la tige de liaison 12 dans l'orifice de passage 15, le bossage arrondi 26 force le bloc de centrage 116 à un mouvement de translation dans le logement 22. Ainsi, lorsque la tige de liaison 12 s'éloigne de la position prédéfinie représentée à la Figure 3a, le bossage arrondi 26, compte tenu de la forme de l'encoche sphérique 118, pousse le bloc de centrage 116 de manière à comprimer le ressort hélicoïdal 117 en réduisant l'espace 23 entre le bloc de centrage 116 et le fond du logement 22, tel que représenté sur la Figure 3b. La compression du ressort hélicoïdal 117 s'oppose alors, dans ce cas, au déplacement relatif de la tige de liaison 12 dans l'orifice de passage 15. Ainsi, lorsque la tige de liaison 12 et/ou l'élément de fixation 100 ne sont plus soumis à une contrainte extérieure qui impliquait leur mouvement relatif, et tant que les première et seconde surfaces de centrage (18, 19) sont en contact, celles-ci glissent l'une sur l'autre de manière à décomprimer le ressort hélicoïdal 117. Un tel glissement a pour effet de ramener la tige de liaison 12 vers la position prédéfinie représentée à la Figure 3a.

Le bloc de centrage 116, poussé par le ressort hélicoïdal 117, exerce une force sur la tige de liaison 12 qui est maintenue plaquée contre la surface supplémentaire de centrage 24. Cette surface supplémentaire de centrage 24, de la même manière que la première surface de centrage 18, participe au guidage de la tige de liaison 12 vers la position prédéfinie de la Figure 3a, grâce à sa forme adaptée et complémentaire du bossage arrondi 26.

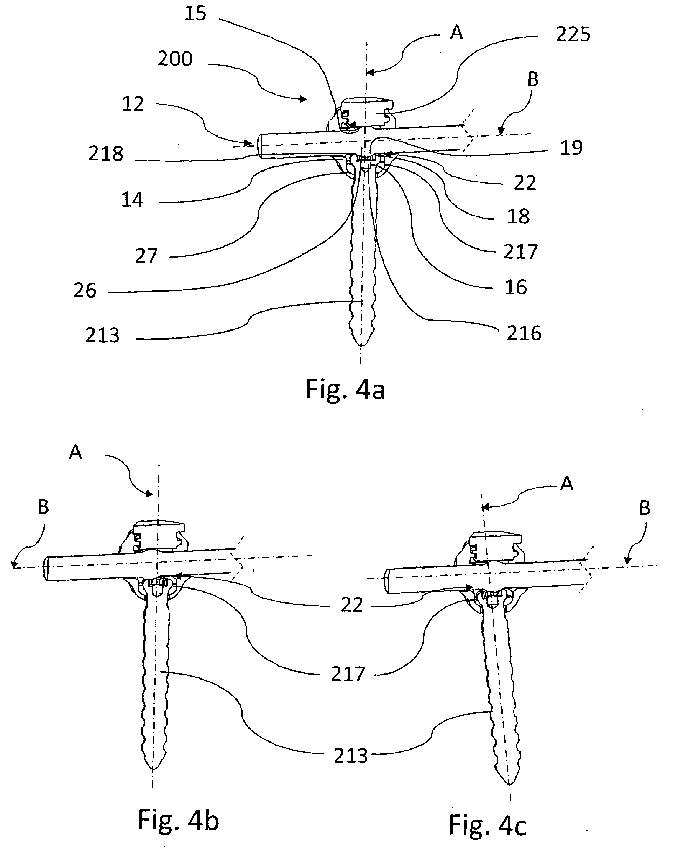

Les Figures 4a, 4b et 4c sont des vues en coupe longitudinale d'un élément de fixation 200 selon un deuxième mode de réalisation de l'invention, dans l'orifice de passage 15 duquel est introduite une tige de liaison 12 semblable à celle déjà décrite sur les Figures 3a et 3b, par exemple. L'élément de fixation 200 présente une tête 14 et un organe d'ancrage 13, comprenant par exemple une vis 213, mobiles l'un par rapport à l'autre en translation et/ou en rotation. Dans ce mode de réalisation, la tête 14 comporte un logement 22, débouchant à l'intérieur de l'orifice de passage 15. Dans ce mode de réalisation, le logement 22 est traversant, selon un axe A sensiblement perpendiculaire à l'axe B de l'orifice de passage 15. Une des ouvertures du logement 22 comporte un rebord 27 et l'autre ouverture est destinée à être obturée par un verrou 225. Dans cet élément de fixation 200, les moyens de centrage 16 sont solidaires de l'organe d'ancrage 13 et forment une des extrémités 216 de ladite vis 213. L'organe d'ancrage 13 et le rebord 27 sont conçus de manière à ce que la vis 213 puisse s'étendre à travers l'ouverture du logement 22 qui comporte ledit rebord 27 et de manière à empêcher le passage des moyens de centrage 16. Selon ce mode de réalisation, les moyens de rappel élastique 17 comportent un joint annulaire 217 élastiquement déformable disposé à l'intérieur du logement 22 et reposant sur le rebord 27, le joint annulaire 217 étant agencé pour être interposé entre ledit rebord 27 et les moyens de centrage 16. Avantageusement, le joint annulaire 217 est ajusté de manière à exercer un couple de rappel en rotation entre l'organe d'ancrage 13 et la tête 14, vers une position d'alignement de ces deux organes selon l'axe transversal A : un tel couple de rappel est dû à l'élasticité du joint 217, coincé entre le logement 22 d'une part et les moyens de centrage 16 d'autre part. Le joint annulaire 217 étant élastiquement déformable, il permet également, en plus d'un mouvement de rotation, un mouvement de translation de l'extrémité 216 de la vis 213 par rapport à la tête 14 lorsqu'il subit un écrasement élastique.

L'extrémité 216 comprend une première surface de centrage 18 constituée, selon ce mode de réalisation, d'un rebord de centrage 218 ménagé à l'extrémité 216 de l'organe d'ancrage 13 destinée à être en regard de l'orifice de passage 15. Lorsque la tige de liaison 12 est introduite dans l'orifice de passage 15, elle peut occuper une position prédéfinie et privilégiée, telle que représentée à la Figure 4a, dans laquelle le rebord de centrage 218 et le bossage arrondi 26 sont en contact, et dans laquelle les parois de l'orifice de passage 15 et/ou l'extrémité du verrou 215 en regard de l'orifice de passage 15 sont également en contact avec le bossage arrondi 26. Dans cette position prédéfinie le joint annulaire 217 ne subit pas d'écrasement dissymétrique par rapport à l'axe A.

Dans le cas d'un déplacement relatif de la tige de liaison 12 et de l'élément de fixation 200, le bossage arrondi 26 en contact avec le rebord de centrage 218 impose au joint annulaire 217 une déformation élastique, visible notamment sur la Figure 4c, qui crée un effort élastique qui tend à ramener la tige de liaison 12 et l'élément de fixation 200 dans la position prédéfinie telle que décrite à la Figure 4a. Le joint annulaire 217 présente l'avantage de permettre une grande liberté de mouvement relatif entre la tête 14, la vis 213 et la tige de liaison 12, en rotation et/ou translation. En particulier, le joint annulaire 217 permet un de la vis 213 et de la tige de liaison 12 tel que représenté à la Figure 4c par les axes A et B.

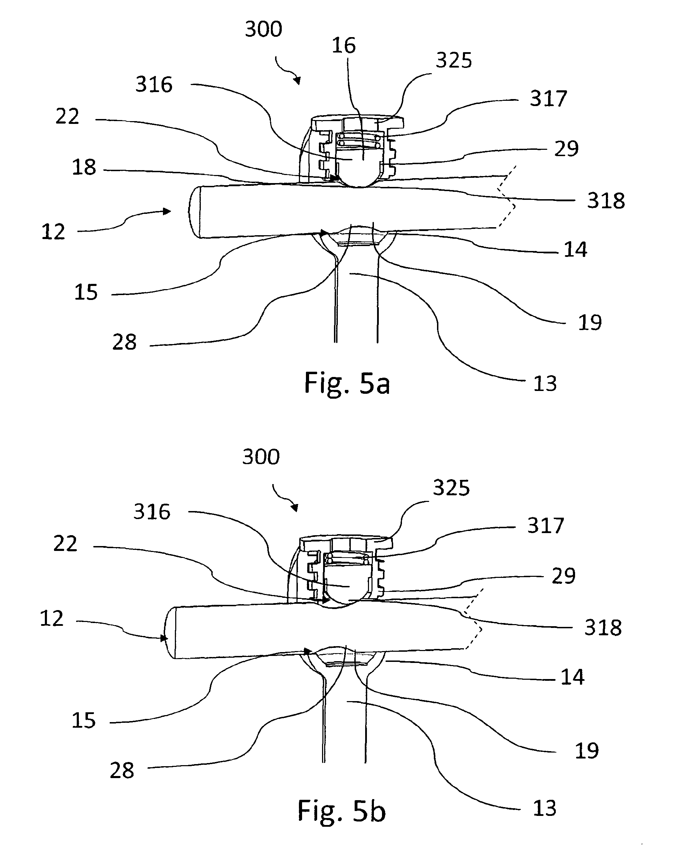

Selon un troisième mode de réalisation, tel que représenté sur les Figures 5a et 5b, un élément de fixation 300 diffère de l'élément de fixation 100 en ce qu'un logement 29 est ménagé au sein d'un verrou 325, ce logement formant une continuité du logement 22 que le verrou 325 est destiné à obturer. Le verrou 325 est, par exemple, fixé à la tête 14 à l'aide d'un pas de vis ménagé dans la paroi du logement 22. Ce logement 29 accueille un ressort hélicoïdal 317 et un bloc de centrage 316 monté sur ledit ressort hélicoïdal 317, le bloc de centrage 316 faisant saillie de ce logement 22 dans l'orifice de passage 15, lorsque le verrou 325 est positionné dans le logement 22. Ce bloc de centrage 316, de même que le bloc de centrage 116 du premier mode de réalisation décrit aux Figures 3a et 3b, constitue les moyens de centrage 16. La première surface de centrage 18 constituant la face du bloc de centrage 316 en regard de l'orifice de passage 15 est par exemple un bossage arrondi 318. Dans ce cas, la seconde surface de centrage 19 ménagée dans la tige de liaison 12 adaptée à cet élément de fixation 300 est une encoche arrondie 28. De même que pour l'élément de fixation 100 du premier mode de réalisation, un déplacement relatif de la tige de liaison 12 par rapport à la tête 14 de l'élément de fixation 300 implique une compression du ressort hélicoïdal 317, comme le montre la Figure 5b, qui exerce alors une force sur le bloc de centrage 316, transmise à la tige de liaison 12 par le contact glissant entre la première surface de centrage 18 et la seconde surface de centrage 19. Cette force tend à ramener la tige de liaison 12 dans la position prédéfinie telle que représentée sur la Figure 5a.

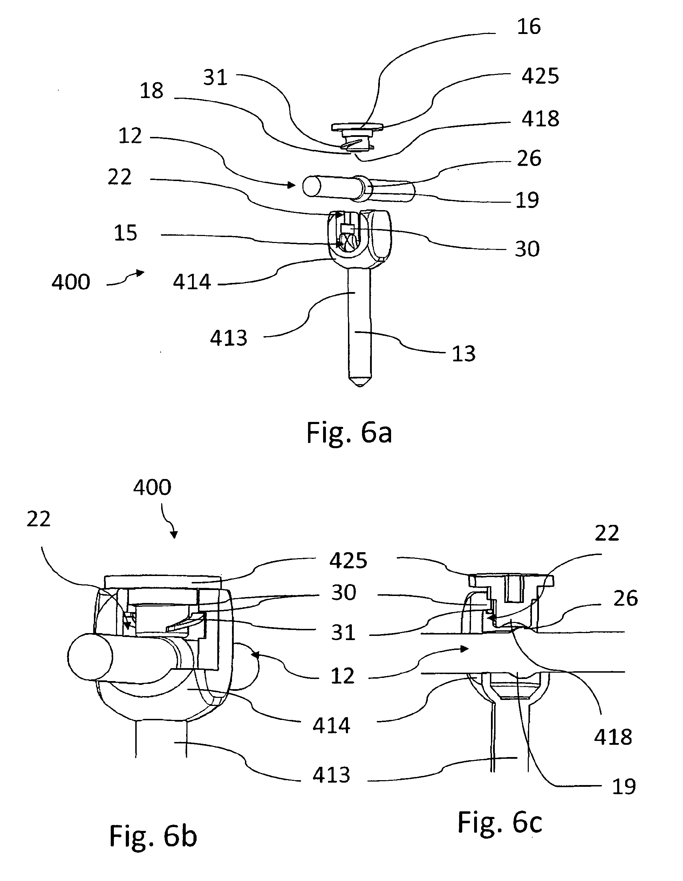

Selon un quatrième mode de réalisation, tel que celui représenté sur les Figures 6a à 6c, un élément de fixation 400 comporte un organe d'ancrage 13 constitué d'une vis 413 prolongée d'une tête 414 en forme de « U », l'espace situé entre les branches du « U » constituant le logement 22 et l'orifice de passage 15. Dans ce mode de réalisation, le logement 22 comporte au moins une butée 30 ménagée dans la surface dudit logement 22, c'est-à-dire sur les branches du « U ». L'élément de fixation 400, représenté sur les Figures 6a à 6c, comporte deux butées 30 disposées en regard l'une de l'autre et faisant saillie dans le logement 22. Selon une variante non représentée de l'invention, une butée est par exemple une portion de pas de vis.

Un verrou 425 destiné à obturer le logement 22 comporte au moins deux ailettes 31 souples destinées à coopérer avec lesdites butées 30 afin de permettre la fixation du verrou 425 sur la tête 414. Le verrou 425 présente une première surface de centrage 18 constituée d'une encoche arrondie 418, visible sur la Figure 6c, située sur la face du verrou 425 destinée à être placée en regard de l'orifice de passage 15.

L'assemblage de l'implant 10 réalisé à l'aide d'un élément de fixation 400 est particulièrement aisé puisque une tige de liaison 12, telle que celle décrite précédemment avec les premier et deuxième modes de réalisation de l'élément de fixation (100, 200), est insérée entre les branches de la tête 414 en forme de « U », comme le montre la Figure 6a, puis le verrou 425 est vissé sur la tête 414 pour amener les ailettes 31 à coopérer avec les butées 30 afin de retenir la tige de liaison 12. Une fois le verrou 425 positionné et retenu par les ailettes 31 et les butées 30, les première et seconde surface de centrage (18, 19) assurent le centrage à l'aide des ailettes 31. Celles-ci assurent la fonction des moyens de rappel élastique 16 grâce à leur souplesse, en plus de leur fonction de fixation du verrou 425. Ainsi, lors d'un déplacement relatif de la tige de liaison 12 par rapport à l'élément de fixation 400, le glissement du bossage arrondi 26 suivant l'encoche arrondie 418 provoque un déplacement du verrou 425. Ce déplacement du verrou s'effectue suivant un axe parallèle aux branches du « U » de la tête 414 grâce à une déformation élastique des ailettes 31, comme le montre la Figure 6c. Ces déformations élastiques des ailettes 31 créent une force de rappel élastique s'opposant au déplacement dudit verrou 425 et de la tige de liaison 12 tendant à ramener la dite tige de liaison 12 par glissement des première et deuxième surfaces de centrage (18, 19) dans une position prédéfinie décrite aux Figures 6a et 6b. Dans cette position prédéfinie, les ailettes 31 ne subissent aucune déformation élastique.

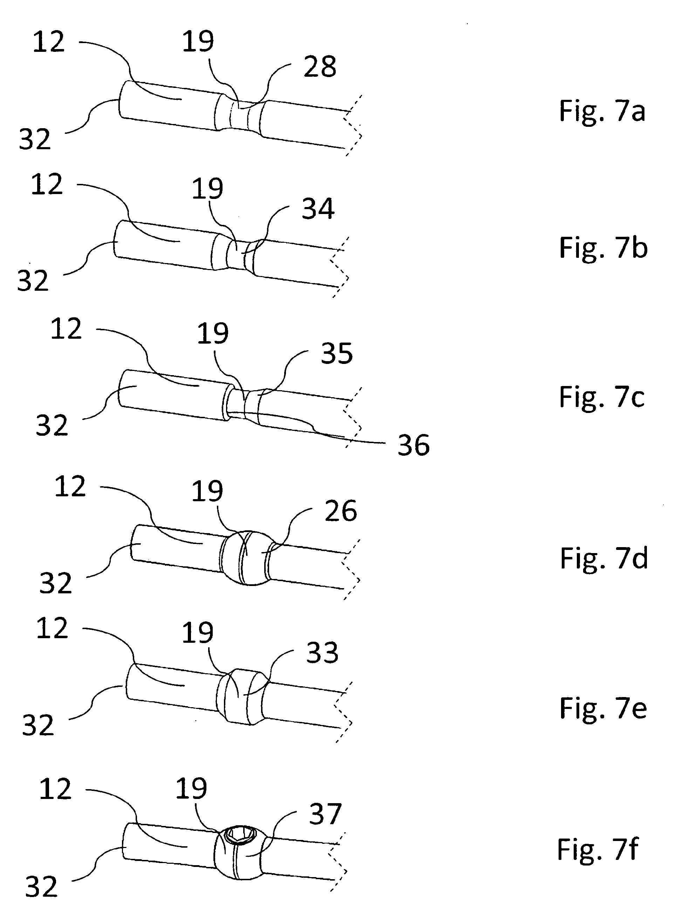

La tige de liaison 12 selon l'invention, utilisée en coopération avec un élément de fixation 100, 200, 300, 400 selon l'invention présente à proximité de l'une 32 de ses extrémités une seconde surface de centrage 19 telle que un bossage arrondie 26, décrit sur les Figures 3a, 3b, 4a, 4b, 4c, 6a, 6b et 6c ou encore une encoche arrondie 28 telle que représentée sur les Figures 5a et 5b. La seconde surface de centrage 19 n'est cependant pas limitée à ces formes et peut, par exemple, prendre diverses formes telles que celles représentées sur les Figures 7a à 7f. L'homme du métier saura alors adapter la forme de la première surface de centrage pour assurer une coopération adéquate de la tige de liaison et de l'élément de fixation. On retrouve le bossage arrondi 26 représenté à la Figure 7d et l'encoche arrondie 28 à la Figure 7a. Un bossage peut également présenter des pentes non incurvées telles que, par exemple, le bossage 33 de la Figure 7e. De même une encoche peut comporter des pentes non arrondies telles que celles de l'encoche 34 représentée à la Figure 7b. Par ailleurs, l'encoche ou le bossage peut être asymétrique telle que l'encoche 35 représentée sur la Figure 7c, qui présente une paroi 36 formant butée. Une telle encoche 35 peut être intéressante pour empêcher le déplacement relatif dans une direction de la tige de liaison 12 avec l'élément de fixation associé, grâce à la paroi 36 formant butée : la paroi 36 formant butée empêche le déplacement de l'élément de fixation vers l'extrémité 32 de ladite tige de liaison 12.

Par ailleurs, la seconde surface de centrage 19 peut également être formée par une bague 37 rapportée sur la tige de liaison 12 selon l'invention comme c'est le cas de la tige de liaison 12 représentée sur la Figure 7f.

Bien entendu les exemples de réalisations évoqués ci-dessus ne présentent aucun caractère limitatif et d'autres détails et améliorations peuvent être apportés à un élément de fixation 100, 200, 300, 400 ou à une tige de liaison 12 selon l'invention, sans pour autant sortir du cadre de l'invention où d'autres formes d'un élément de fixation ou d'une tige de liaison peuvent être réalisées : notamment, outre les formes détaillées dans les Figures, les première et deuxième surfaces de centrage (18, 19) peuvent comporter des éléments de centrage en rotation, non représentés, aptes à imprimer un mouvement de rotation afin de ramener la tige de liaison dans une position prédéfinie.

## Revendications

1. Elément de fixation (100, 200, 300, 400) pour un implant (10) de maintien d'une partie d'un rachis de l'homme ou de l'animal, comprenant, d'une part, un organe d'ancrage (13) destiné à assurer la fixation de l'élément dans un corps vertébral (3) et, d'autre part, une tête (14) dans laquelle est ménagé un orifice de passage (15) conformé pour recevoir une tige de liaison (12), **caractérisé en ce qu'**il comporte des moyens de centrage (16) de ladite tige de liaison (12) montés sur des moyens de rappel élastique (17) disposés dans ladite tête (14), lesdits moyens de centrage (16) et ladite tête (14) étant mobiles l'un par rapport à l'autre.

2. Elément de fixation (100, 200, 300, 400) selon la revendication 1, dans lequel le mouvement relatif des moyens de centrage (16) s'effectue selon un axe (A) transversal, notamment perpendiculaire, à l'axe (B) de l'orifice de passage (15).

3. Elément de fixation (100, 200, 300, 400) selon la revendication 1 ou 2, dans lequel lesdits moyens de rappel élastique (17) tendent à pousser lesdits moyens de centrage (16) vers l'intérieur de l'orifice de passage (15).

4. Elément de fixation (100, 200, 300, 400) selon l'une des revendications 1 à 3, dans lequel ladite tête (14) comporte en outre un logement (22) débouchant dans l'orifice de passage (15) à l'intérieur duquel sont ménagés les moyens de rappel élastique (17) et les moyens de centrage (16).

5. Elément de fixation (300, 400) selon la revendication 2, comprenant en outre un verrou (325, 425) destiné à obturer le logement (22) réalisé débouchant en outre à l'extérieur de ladite tête, le verrou (325, 425) comprenant les moyens de rappel élastique (317, 31) et les moyens de centrage (318, 418).

6. Elément de fixation (400) selon la revendication 5, dans lequel le verrou (425) comporte des ailettes (31) souples destinées à coopérer avec au moins une butée (30) ménagée dans la surface du logement (22).

7. Elément de fixation (200) selon la revendication 2, **caractérisé en ce que** les moyens de centrage (16) sont solidaires de l'organe d'ancrage (213), lesdits moyens de rappel élastique (217) étant montés entre la tête (14) et ledit organe d'ancrage (213).

8. Elément de fixation (200) selon la revendication 7, dans lequel la tête (14) et l'organe d'ancrage (213) sont mobiles en translation et/ou en rotation l'un par rapport à l'autre.

9. Elément de fixation (200) selon la revendication 8, dans lequel les moyens de rappel élastique (217) sont conçus de manière à exercer un couple de rappel en rotation entre la tête (14) et l'organe d'ancrage (213).

10. Elément de fixation (100, 200, 300, 400) selon l'une des revendications 1 à 9, dans lequel ledit moyen de centrage (16) comporte une première surface de centrage (18) en regard de l'orifice de passage (15) présentant au moins une pente, notamment de forme plane ou incurvée.

11. Elément de fixation (100, 200, 400) selon la revendication 10, dans lequel la première surface de centrage (18) comporte une encoche (118, 218, 418).

12. Elément de fixation (300) selon la revendication 10, dans lequel la première surface de centrage (18) comporte un bossage (318).

13. Elément de fixation (100) selon l'une des revendications 1 à 12, dans lequel au moins une partie de la surface délimitant l'orifice de passage (15) présente une surface de centrage (24) supplémentaire comportant un bossage et/ou une encoche.

14. Tige de liaison (12) pour un implant (10) de maintien du rachis de l'homme ou de l'animal, apte à être insérée dans un orifice de passage (15) d'un élément de fixation (100, 200, 300, 400) selon l'une des revendications précédentes et comprenant au moins une surface de centrage, dite seconde surface de centrage (19), comportant une encoche (28, 34, 35) et/ou une saillie (26, 33, 37), ladite seconde surface de centrage (19) étant apte à coopérer avec la première surface de centrage (18) des moyens de centrage (16).

15. Tige de liaison (12) selon la revendication 14, dans laquelle chaque seconde surface de centrage (19) est ménagée à proximité d'une (32) des extrémités de la tige de liaison (12).

16. Implant (10) pour la stabilisation d'un rachis pour le corps humain ou animal comportant au moins un élément de fixation (100, 200, 300, 400) selon l'une des revendications 1 à 13 et au moins une tige de liaison (12) selon la revendication 14 ou 15 insérée dans l'orifice de passage (15) dudit élément de fixation, les moyens de centrage (16) étant conçus pour coopérer avec ladite seconde surface de centrage (19).

17. Implant (10) selon la revendication 16 en combinaison avec la revendication 10, dans lequel ladite tige de liaison (12) est mobile en rotation et/ou en translation par rapport à l'organe d'ancrage (13), lesdites première et seconde surfaces de centrage (18, 19) étant conçues pour coopérer afin de déplacer ladite tige de liaison (12) en rotation et/ou en translation par rapport à l'organe d'ancrage (13).

## Claims

1. An attachment element (100, 200, 300, 400) for a maintenance implant (10) of part of a human or animal vertebral column, comprising, on the one hand, an anchor member (13) intended to attach the element in a vertebral body (3) and, on the other hand, a head (14) in which a passage opening (15) is formed conducive to receiving a connecting rod (12), **characterized in that** it includes a means (16) for centering said connecting rod (12) mounted on a spring-back means (17) arranged in said head (14), said centering means (16) and said head (14) being mobile relative to each other.

2. The attachment element (100, 200, 300, 400) according to claim 1, wherein the relative movement of the centering means (16) is done along an axis (A) transverse, in particular perpendicular, to the axis (B) of the passage opening (15).

3. The attachment element (100, 200, 300, 400) according to claim 1 or 2, wherein said spring-back means (17) tend to push said centering means (16) towards the inside of the passage opening (15).

4. The attachment element (100, 200, 300, 400) according to one of claims 1 to 3, wherein said head (14) also has a housing (22) emerging in the passage opening (15) inside which the spring-back means (17) and the centering means (16) are housed.

5. The attachment element (300, 400) according to claim 2, also comprising a bolt (325, 425) intended to close the housing (22) made to emerge outside said head, the bolt (325, 425) comprising the spring-back means (317, 31) and the centering means (318, 418).

6. The attachment element (400) according to claim 5, wherein the bolt (425) has flexible fins (31) intended to cooperate with a stop (30) formed in the surface of the housing (22).

7. The attachment element (200) according to claim 2, **characterized in that** the centering means (16) is secured to the anchor member (213), said spring-back means (217) being mounted between the head (14) and said anchor member (213).

8. The attachment element (200) according to claim 7, wherein the head (14) and the anchor member (213) are translationally and/or rotationally mobile relative to each other.

9. The attachment element (200) according to claim 8, wherein the spring-back means (217) is designed so as to exert a rotational return torque between the head (14) and the anchor member (213).

10. The attachment element (100, 200, 300, 400) according to one of claims 1 to 9, wherein said centering means (16) includes a first centering surface (18) opposite the passage opening (15) having at least one slope, in particular planar or curved.

11. The attachment element (100, 200, 400) according to claim 10, wherein the first centering surface (18) has a notch (118, 218, 418).

12. The attachment element (300) according to claim 10, wherein the first centering surface (18) has a boss (318).

13. The attachment element (100) according to one of claims 1 to 12, wherein at least part of the surface delimiting the passage opening (15) has an additional centering surface (24) having a boss and/or a notch.

14. A connecting rod (12) for a maintenance implant (10) for a human or animal vertebral column, able to be inserted into a passage opening (15) of an attachment element (100, 200, 300, 400) according to one of the preceding claims and comprising at least one centering surface, called second centering surface (19), including a notch (28, 34, 35) and/or a protrusion (26, 33, 37), said second surface (19) being able to cooperate with the first centering surface (18) of the centering means (16).

15. The connecting rod (12) according to claim 14, wherein each second surface (19) is formed near one (32) of the ends of the connecting rod (12).

16. An implant (10) for stabilizing a vertebral column in the human or animal body having at least one attachment element (100, 200, 300, 400) according to one of claims 1 to 13 and at least one connecting rod (12) according to one of claims 14 or 15, inserted into the passage opening (15) of said attachment element, the centering means (16) being designed to cooperate with said second centering surface (19).

17. The implant (10) according to claim 16 combined with claim 10, wherein said connecting rod (12) is rotationally and/or translationally mobile relative to the anchor member (13), said first and second centering surfaces (18, 19) being designed to cooperate so as to move said connecting rod (12) rotationally and/or translationally relative to the anchor member (13).

## Patentansprüche

1. Befestigungselement (100, 200, 300, 400) für ein Implantat (10) zum Halten eines Abschnitts eines Wirbels eines Menschen oder Tiers, das einerseits ein Verankerungsorgan (13) umfasst, das dazu bestimmt ist, die Befestigung des Elements in einem Wirbelkörper (3) sicherzustellen, und andererseits einen Kopf (14), in den eine Durchgangsöffnung (15) eingearbeitet ist, die ausgebildet ist, um eine Verbindungsstange (12) aufzunehmen, **dadurch gekennzeichnet, dass** es Zentriermittel (16) der Verbindungsstange (12) aufweist, die auf elastischen Rückstellmitteln (17) montiert sind, die in dem Kopf (14) angeordnet sind, wobei die Zentriermittel (16) und der Kopf (14) im Verhältnis zueinander bewegbar sind.

2. Befestigungselement (100, 200, 300, 400) nach Anspruch 1, wobei die relative Bewegung der Zentriermittel (16) gemäß einer transversalen Achse (A), insbesondere senkrecht, zur Achse (B) der Durchgangsöffnung (15), erfolgt.

3. Befestigungselement (100, 200, 300, 400) nach Anspruch 1 oder 2, wobei die elastischen Rückstellmittel (17) dazu neigen, die Zentriermittel (16) in das Innere der Durchgangsöffnung (15) zu drücken.

4. Befestigungselement (100, 200, 300, 400) nach einem der Ansprüche 1 bis 3, wobei der Kopf (14) ferner eine Aufnahme (22) aufweist, die in die Durchgangsöffnung (15) mündet, in der die elastischen Rückstellmittel (17) und die Zentriermittel (16) eingearbeitet sind.

5. Befestigungselement (300, 400) nach Anspruch 2, das ferner einen Riegel (325, 425) umfasst, der dazu bestimmt ist, die Aufnahme (22) zu verschließen, die ferner außerhalb des Kopfes mündend realisiert ist, wobei der Riegel (325, 425) die elastischen Rückstellmittel (317, 31) und die Zentriermittel (318, 418) umfasst.

6. Befestigungselement (400) nach Anspruch 5, wobei der Riegel (425) elastische Flügel (31) aufweist, die dazu bestimmt sind, mit mindestens einem Anschlag (30) zusammenzuarbeiten, der in die Fläche der Aufnahme (22) eingearbeitet ist.

7. Befestigungselement (200) nach Anspruch 2, **dadurch gekennzeichnet, dass** die Zentriermittel (16) mit dem Verankerungsorgan (213) verbunden sind, wobei die elastischen Rückstellmittel (217) zwischen dem Kopf (14) und dem Verankerungsorgan (213) montiert sind.

8. Befestigungselement (200) nach Anspruch 7, wobei der Kopf (14) und das Verankerungsorgan (213) im Verhältnis zueinander verschiebend und/oder rotierend bewegbar sind.

9. Befestigungselement (200) nach Anspruch 8, wobei die elastischen Rückstellmittel (217) derart konstruiert sind, dass eine rotierende Rückstellkraft zwischen dem Kopf (14) und dem Verankerungsorgan (213) ausgeübt wird.

10. Befestigungselement (100, 200, 300, 400) nach einem der Ansprüche 1 bis 9, wobei das Zentriermittel (16) eine erste Zentrierfläche (18) gegenüber der Durchgangsöffnung (15) aufweist, die mindestens eine, insbesondere ebene oder gekrümmte, Neigung aufweist.

11. Befestigungselement (100, 200, 400) nach Anspruch 10, wobei die erste Zentrierfläche (18) eine Kerbe (118, 218, 418) aufweist.

12. Befestigungselement (300) nach Anspruch 10, wobei die erste Zentrierfläche (18) eine Wulst (318) aufweist.

13. Befestigungselement (100) nach einem der Ansprüche 1 bis 12, wobei mindestens ein Abschnitt der Fläche, die die Durchgangsöffnung (15) begrenzt, eine zusätzliche Zentrierfläche (24) aufweist, die eine Wulst und/oder eine Kerbe aufweist.

14. Verbindungsstange (12) für ein Implantat (10) zum Halten des Wirbels eines Menschen oder Tiers, die imstande ist, in eine Durchgangsöffnung (15) eines Befestigungselements (100, 200, 300, 400) nach einem der vorangehenden Ansprüche eingeführt zu sein und mindestens eine als zweite Zentrierfläche (19) bezeichnete Zentrierfläche umfasst, die eine Kerbe (28, 34, 35) und/oder einen Vorsprung (26, 33, 37) aufweist, wobei die zweite Zentrierfläche (19) imstande ist, mit der ersten Zentrierfläche (18) der Zentriermittel (16) zusammenzuarbeiten.

15. Verbindungsstange (12) nach Anspruch 14, wobei jede zweite Zentrierfläche (19) in der Nähe eines (32) der Enden der Verbindungsstange (12) ausgebildet ist.

16. Implantat (10) zur Stabilisierung eines Wirbels für den Körper eines Menschen oder Tiers, das mindestens ein Befestigungselement (100, 200, 300, 400) nach einem der Ansprüche 1 bis 13 und mindestens eine Verbindungsstange (12) nach Anspruch 14 oder 15 aufweist, die in die Durchgangsöffnung (15) des Befestigungselements eingeführt ist, wobei die Zentriermittel (16) ausgebildet sind, um mit der zweiten Zentrierfläche (19) zusammenzuarbeiten.

17. Implantat (10) nach Anspruch 16 in Kombination mit Anspruch 10, wobei die Verbindungsstange (12) im Verhältnis zum Verankerungsorgan (13) rotierend und/oder verschiebend bewegbar ist, wobei die erste und zweite Zentrierfläche (18, 19) ausgebildet sind, um zusammenzuarbeiten, um die Verbindungsstange (12) rotierend und/oder verschiebend im Verhältnis zum Verankerungsorgan (13) zu versetzen.
